# EUROPÄISCHE PATENTSCHRIFT

(11) **EP 2 584 336 B1**
(45) Veröffentlichungstag und Bekanntmachung des Hinweises auf die Patenterteilung: **20.02.2019**
(21) Anmeldenummer: 12187249.3
(22) Anmeldetag: 04.10.2012
(51) Int. Cl.: G01N 1/12, G01N 33/20, B22D 2/00

(54) **Vorrichtung zum Messen von Parametern oder zur Probennahme in Eisen- oder Stahlschmelzen**
Device for measuring parameters or for sampling in iron or steel melts
Dispositif destiné à mesurer des paramètres ou à prélever des échantillons dans l'acier ou le fer en fusion

(30) Priorität: 20.10.2011 DE 102011116440
(43) Veröffentlichungstag der Anmeldung: 24.04.2013
(73) Patentinhaber: Heraeus Electro-Nite International N.V., 3530 Houthalen (BE)
(72) Erfinder: NEYENS, Guido Jacobus, 3680 Opoeteren (BE); BORTELS, Eric B., 3560 Lummen (BE); BEYENS, Dries, 3640 Kinrooi (BE)
(74) Vertreter: Heraeus IP

(56) Entgegenhaltungen:
- EP-A2- 1 813 940
- WO-A1-89/11637
- DD-A5- 214 441
- DE-A1- 3 000 201
- DE-A1- 3 402 818
- DE-A1- 3 907 900
- DE-A1- 4 303 688
- DE-A1-102009 059 780
- DE-B4- 19 758 595
- DE-T2- 69 925 388
- GB-A- 1 150 149
- US-A- 3 656 350
- US-A- 4 237 734
- US-A- 4 261 202
- US-A- 4 842 418

## Beschreibung

Die Erfindung betrifft eine Vorrichtung zum Messen von Parametern oder zur Probennahme in Eisen- oder Stahlschmelzen sowie zur Probennahme von auf einer Eisen- oder Stahlschmelze aufliegenden Schlacke, mit einem ein Eintauchende und eine seitliche Umfangsfläche aufweisenden Trägerrohr, an dessen Eintauchende ein Eintauchende und eine seitliche Umfangsfläche aufweisender Messkopf angeordnet ist, wobei an dem Eintauchende des Messkopfes mindestens ein Sensor oder eine Einlauföffnung für eine im Inneren der Vorrichtung vorhandene Probenkammer angeordnet ist.

Derartige Vorrichtungen sind beispielsweise aus DE 197 58 595 B4 bekannt. Hier sind Vorrichtungen beschrieben, an deren Stirnseite sowohl ein Thermoelement als auch eine Einlauföffnung für eine Probenkammer angeordnet sind. Die Probenkammer ist zur Entnahme von Schlackeproben geeignet. Darüberhinaus ist an der Stirnseite dieser Vorrichtung ein weiterer Einlauf für eine Probenkammer angeordnet, die zur Entnahme von Metallschmelzenproben geeignet ist. Eine weitere aus dieser Druckschrift bekannte Vorrichtung weist einen Probennehmer mit zwei seitlichen Einlauföffnungen auf.

Ähnliche Probennehmer sind aus DE 197 52 743 C5 bekannt. Ein Probennehmer mit einer Einlauföffnung an einer seitlichen Umfangsfläche eines Trägerrohres ist aus der DE 699 25 388 T2 bekannt. Probennehmer für Schlackenproben sind zudem aus EP 1 183 513 B1 bekannt. Diese entsprechen im Wesentlichen den bereits aus DE 197 58 595 B4 bekannten Probennehmern.

Probennehmer für Metallschmelzen, bei denen sich Schlacke und andere nichtmetallische Einschlüsse aus flüssigem Metall absetzen sind beispielsweise aus DE 41 29 930 A1 oder aus US 5,415,052 oder US 5,515,739 bekannt. Hier wird jeweils in einer der Probenkammer für Metallschmelzen vorgelagerten Vorkammer Verunreinigung gesammelt, die beim Einfließen der Metallschmelze in die Probenkammer von der Metallschmelze separiert werden soll, um die Qualität der Metallproben zu verbessern.

Aufgabe der vorliegenden Erfindung ist es, Probennehmer, insbesondere zur Entnahme von Schlackenproben zu verbessern und qualitativ hochwertige und eine genaue Analyse ermöglichende Proben zur Verfügung zu stellen.

Die Aufgabe wird durch die Merkmale des Anspruchs 1 gelöst. Vorteilhafte Ausgestaltungen sind in den abhängigen Ansprüchen angegeben. Insbesondere ist die Erfindung dadurch gekennzeichnet, dass an der seitlichen Umfangsfläche des Trägerrohres eine metallische Spritzschutzschicht angeordnet ist und die Spritzschutzschicht eine an der seitlichen Umfangsfläche des Trägerrohres angeordnete Einlauföffnung umgibt.

Vorteilhaft schützt die Spritzschutzschicht das Material des Trägerrohres in der unmittelbaren Umgebung der Einlauföffnung und verhindert damit, dass beim Eintauchen des Trägerrohres in die Schlackeschicht Teile des Trägerrohres oder deren Verbrennungsprodukte in die Vorkammer und anschließend in die Probenkammer gelangen und die Probe verfälschen. Dadurch wird die Probenqualität verbessert. Vorzugsweise umfasst die Spritzschutzschicht das Trägerrohr von ihrem Eintauchende bis oberhalb der an der seitlichen Umfangsfläche des Trägerrohres angeordneten Einlauföffnung.

Die Spritzschutzschicht kann relativ dünn ausgebildet sein, beispielsweise mit einer Dicke von etwa 0,5 Millimeter. Dies ist vorteilhaft, damit die Spritzschutzschicht während des Eindringens der Vorrichtung in die Schlackenschicht das Trägerrohr schützt, so dass eine qualitativ hochwertige Füllung der Probenkammer mit Schlacke gewährleistet ist. Danach kann sich die Spritzschutzschicht zweckmäßigerweise auflösen, damit nach dem Herausziehen der Vorrichtung aus der Schmelze eine leichtere Entnahme der Probenkammer mit der Probe aus der Vorrichtung möglich ist.

Die Spritzschutzschicht kann vorteilhafterweise rohrförmig ausgebildet sein, wobei sie an der Außenwand des Trägerrohres umlaufend anliegt. Die Spritzschutzschicht kann insbesondere eine von dem Trägerrohr abgewandte, also radial nach außen weisende Oberfläche von mindestens 250 Quadratzentimeter aufweisen. Dies ist beispielsweise vorteilhaft bei herkömmlichen Trägerrohren mit einem Durchmesser von etwa 80 Millimeter.

Weiterhin sind mit der erfindungsgemäßen Vorrichtung eine Messung oder Probennahme an der in die Stahlschmelze tief eintauchenden Stirnseite der Vorrichtung möglich, sowie eine Probenentnahme aus einem höher gelegenen Ort, der im Bereich einer auf der Stahlschmelze aufliegenden Schlackeschicht liegt. Hier wird die Schlacke zunächst in eine Vorkammer und dann in eine Probenkammer geleitet, wobei die Schlacke, weil sie leichter als die Stahlschmelze ist, aufsteigt und schwerere, eventuell in die Vorkammer mit eindringende Metallschmelzenanteile in der Vorkammer verbleiben. Dadurch können hochwertige Schlackenproben gewonnen werden.

Da die Eintauchtiefe einerseits und die Dicke der auf der Stahlschmelze aufliegenden Schlackeschicht andererseits relativ genau bekannt sind, lässt sich die Eintauchtiefe der Vorrichtung sehr genau so steuern, dass die Stirnseite in der Metallschmelze angeordnet wird, während die seitliche Einlauföffnung in der Schlackeschicht angeordnet ist.

Ein Messkopf ist im allgemeinen bei den hier beschriebenen Eintauchsonden ein an dem Eintauchende eines Trägerrohres angeordnetes separates Bauteil, an oder in dem Sensoren oder Probenkammern angeordnet sind. Derartige Messköpfe sind meist im Wesentlichen aus Metall, insbesondere aus Stahl oder aus Gießereisand oder Zement gebildet. Trägerrohre sind im allgemeinen aus Pappe gebildet und werden auf sogenannte Lanzen aufgesteckt, die entweder automatisch oder manuell bedient werden und mit denen die Trägerrohre mit dem Messkopf in die Schmelze eingetaucht werden. Die Lanzen sind für eine Mehrfachverwendung geeignet, während die Trägerrohre mit dem Messkopf nach einer Messung verbraucht sind und ersetzt werden müssen.

Vorteilhaft ist es, dass der Abstand zwischen dem Eintauchende des Messkopfes und der an der seitlichen Umfangsfläche des Trägerrohres oder Messkopfes angeordneten Einlauföffnung kleiner als 50 Zentimeter ist. Des Weiteren ist es vorteilhaft, dass dieser Abstand größer als 15 Zentimeter ist. Dadurch erfolgt eine sehr sichere Platzierung der seitlich angeordneten Einlauföffnung in der Schlackenschicht bei gleichzeitig ausreichender Eintauchtiefe des Eintauchendes des Messkopfes in die Eisen- oder Stahlschmelze.

Das Volumen der Vorkammer ist zweckmäßigerweise größer als das Volumen der Schlackenprobenkammer, so dass eine ausreichende Trennung der Schlacke von dem gesamt einströmenden Material möglich ist und die Schlackenprobe in der Probenkammer eine optimale Qualität aufweist. Dabei kann es vorteilhaft sein, dass die Vorkammer etwa doppelt so groß ist wie die Probenkammer. Vorteilhaft ist es weiterhin, dass der Durchmesser der an der seitlichen Umfangsfläche angeordneten Einlauföffnung in die Vorkammer größer ist als der Durchmesser der in die Schlackenprobenkammer mündenden Eingangsöffnung. Die an der seitlichen Umfangsfläche angeordnete Einlauföffnung kann vorteilhafterweise von einem brennbaren Material, insbesondere von Papier oder Pappe abgedeckt sein.

Des Weiteren ist es vorteilhaft, dass die Schlackenprobenkammer an ihrem der Vorkammer zugewandten Ende und an ihrem der Vorkammer abgewandten Ende von Metallplatten begrenzt ist, da dies zum einen den Abkühlprozess der Probe begünstigt und zum anderen eine glatte Probenoberfläche erzeugt, die für die Analyse verwendet werden kann.

Vorteilhaft ist es weiterhin, dass die Schlackenprobenkammer zwischen ihrem der Vorkammer zugewandten Ende und ihrem der Vorkammer abgewandten Ende von einer konisch ausgebildeten Wand begrenzt ist, da dadurch eine leichtere Entnahme der Probe aus der Probenkammer möglich ist.

Nachfolgend wird die Erfindung beispielhaft anhand von Zeichnungen näher erläutert.

In den Zeichnungen zeigt
- Figur 1: das Eintauchende einer erfindungsgemäßen Vorrichtung
- Figur 2: eine weitere Ausführungsform der erfindungsgemäßen Vorrichtung
- Figur 3: das Eintauchende der erfindungsgemäßen Vorrichtung im Schnitt.

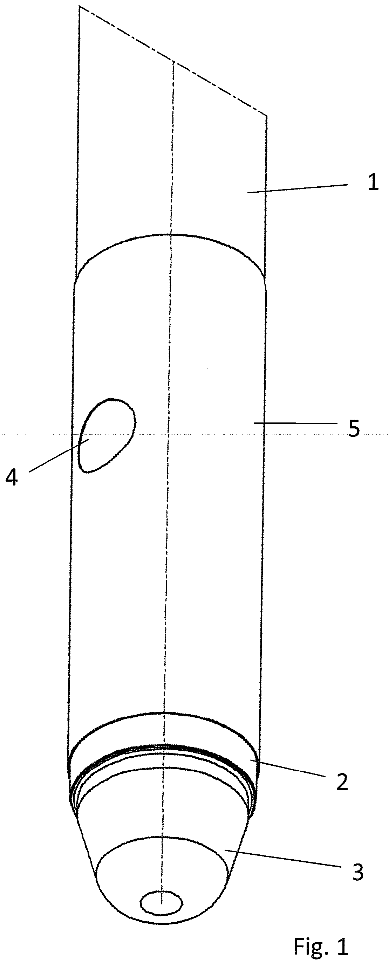

In der in Figur 1 dargestellten Ausführungsform ist ein Trägerrohr 1 aus Pappe zur Halterung eines Messkopfes 2 vorgesehen. Der Messkopf 2 ist an dem Eintauchende des Trägerrohres 1 angeordnet. Er ist aus Giesereisand oder Zement gebildet. Sein Eintauchende ist mit einer Schutzkappe 3 versehen, die die am Messkopf 2 angeordneten Sensoren oder Probenkammern beim Transport und beim Eintauchen durch die Schlackeschicht schützt. Die Schutzkappe 3 ist aus Stahl gebildet. Oberhalb des Messkopfes 2, in einer Entfernung von etwa 20 - 25 Zentimeter vom Eintauchende (der Schutzkappe 3) ist seitlich in dem Trägerrohr 1 eine Einlauföffnung 4 angeordnet.

Das Eintauchende des Trägerrohres 1 ist, beginnend vom Messkopf 2 bis einige Zentimeter oberhalb der Einlauföffnung 4 von einer etwa 0,5 Millimeter dicken Spritzschutzschicht 5 umgeben. Die Spritzschutzschicht 5 kann aus Stahl gebildet sein. Die Einlauföffnung 4 ist an ihrer Außenseite von einer Schicht aus Pappe abgedeckt.

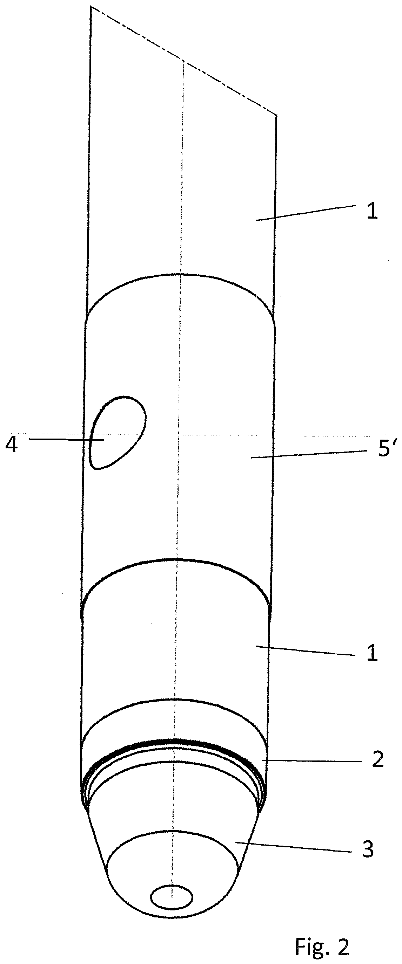

In Figur 2 ist eine ähnliche Anordnung dargestellt, wobei die Spritzschutzschicht 5' nicht bereits am Eintauchende des Trägerrohres 1, also unmittelbar am Messkopf 2 beginnt, sondern erst einige Zentimeter unterhalb, also in Eintauchrichtung vor der Einlauföffnung 4 beginnt und sich an dem, dem Eintauchende abgewandten Ende bis einige Zentimeter hinter der Einlauföffnung 4 erstreckt.

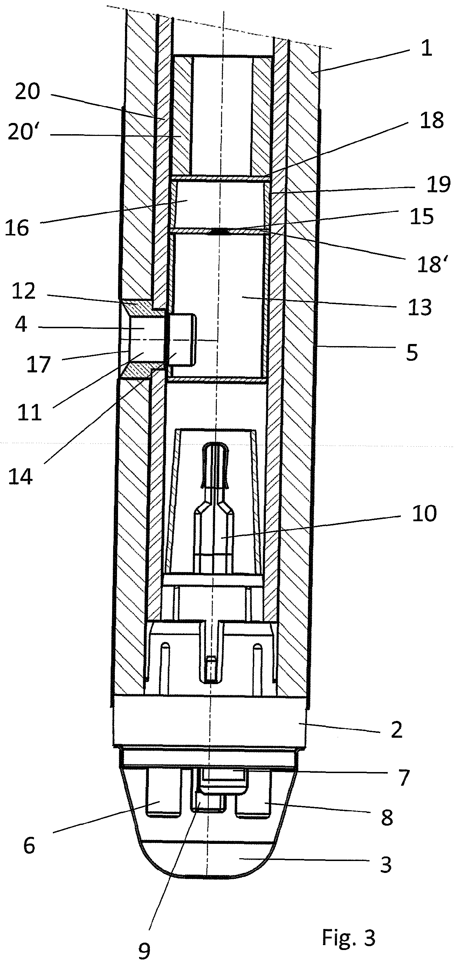

In Figur 3 sind Details einer Ausführungsform einer erfindungsgemäßen Vorrichtung dargestellt. An dem Messkopf 2 ist ein von einer Schutzkappe bedeckter Sauerstoffsensor 6 angeordnet. Der Sauerstoffsensor 6 ist ein elektrochemischer Sensor. Der zugehörige Badkontakt 7 als Gegenelektrode ist ebenfalls an dem Messkopf 2 angeordnet. Des Weiteren trägt der Messkopf 2 ein Thermoelement als Temperatursensor 8, der ebenfalls mit einer Kappe geschützt ist. Zweckmäßigerweise ist an dem Eintauchende des Messkopfes 2 eine Einlauföffnung 9 für eine Probenkammer 10 für Metallschmelze angeordnet.

Die seitliche Einlauföffnung 4 ist aus einem Quarzrohr 11 gebildet, das von Zement 12 in dem Trägerrohr 1 fixiert wird. Das Quarzrohr 11 mündet in die Vorkammer 13 für die Schlackenprobennahme und wird dort von einem Metallhalter 14 gehalten. An ihrem dem Eintauchende abgewandten Ende weist die Vorkammer 13 eine Eingangsöffnung 15 für die Schlackenprobenkammer 16 auf. Die Einlauföffnung 4 hat einen etwa dreimal so großen Durchmesser wie die Eingangsöffnung 15. Die Einlauföffnung 4 ist von einer Pappschicht 17 abgedeckt, die die Vorkammer 13 und die Schlackenprobenkammer 16 vor der Probennahme verschließt und das unbeabsichtigte Eindringen von Material in die Vorkammer 13 oder die Schlackenprobenkammer 16 verhindert.

Die Schlackenprobenkammer 16 ist an ihrem Eintauchende und ihrem dem Eintauchende abgewandten Ende von Stahlscheiben 18; 18' begrenzt, ihre seitliche Umfangsfläche 19 ist ebenfalls aus Stahl gebildet. Sie ist leicht konisch ausgebildet, damit die erstarrte Probe leichter entnommen werden kann. Der Durchmesser der Vorkammer 13 sowie der mittlere Durchmesser der Schlackenprobenkammer 16 beträgt etwa 35 Millimeter, die Vorkammer 13 weist ein etwa doppelt so großes Volumen auf wie die Schlackenprobenkammer 16. Das Volumen der Schlackenprobenkammer 16 beträgt etwa 40 Kubikzentimeter, so dass die Schlackenprobe ein Gewicht von 80 Gramm aufweist. In der Regel werden mindestens 40 Gramm für eine Schlackeanalyse benötigt.

Innerhalb des Trägerrohres 1 sind sogenannte innere Papprohre 20; 20' angeordnet, mit deren Hilfe die Schlackenprobenkammer 16, die Vorkammer 13 sowie andere Einbauten fixiert werden. Dadurch ist eine relativ einfache Herstellung der Vorrichtung und genaue Justierung der einzelnen Teile möglich.

## Patentansprüche

1. Vorrichtung zum Messen von Parametern oder zur Probennahme in Eisen- oder Stahlschmelzen sowie zur Probennahme von auf einer Eisen- oder Stahlschmelze aufliegenden Schlacke, mit einem ein Eintauchende und eine seitliche Umfangsfläche aufweisenden Trägerrohr (1), an dessen Eintauchende ein ein Eintauchende und eine seitliche Umfangsfläche aufweisender Messkopf (2) angeordnet ist, wobei an dem Eintauchende des Messkopfes (2) mindestens ein Sensor oder eine Einlauföffnung (4) für eine im Inneren der Vorrichtung vorhandene Probenkammer (10) angeordnet ist, wobei EPan der seitlichen Umfangsfläche des Trägerrohres (1) oder des Messkopfes (2) eine Einlauföffnung (4) angeordnet ist, die über einen Einlaufkanal in eine im Inneren des Trägerrohres (1) oder des Messkopfes (2) angeordnete Vorkammer (13) mündet und dass die Vorkammer (13) an ihrem dem Eintauchende des Messkopfes (2) abgewandten Ende eine Eingangsöffnung (15) aufweist, die in eine auf der dem Eintauchende abgewandten Seite der Vorkammer (13) im Inneren der Vorrichtung angeordnete Schlackeprobenkammer (16) mündet,
**dadurch gekennzeichnet, dass**
an der seitlichen Umfangsfläche des Trägerrohres (1) eine metallische Spritzschutzschicht (5) angeordnet ist und die Spritzschutzschicht (5) die an der seitlichen Umfangsfläche des Trägerrohres (1) angeordnete Einlauföffnung (4) umgibt.

2. Vorrichtung nach Anspruch 1, **dadurch gekennzeichnet, dass** die Spritzschutzschicht (5) rohrförmig ausgebildet ist.

3. Vorrichtung nach Anspruch 1 oder 2, **dadurch gekennzeichnet, dass** die Spritzschutzschicht (5) die an der seitlichen Umfangsfläche des Trägerrohres (1) angeordnete Einlauföffnung (4) nicht abdeckt.

4. Vorrichtung nach einem der Ansprüche 1 bis 3, **dadurch gekennzeichnet, dass** die Spritzschutzschicht (5) eine von dem Trägerrohr (1) abgewandte Oberfläche von mindestens 250 cm2 aufweist.

5. Vorrichtung nach einem der Ansprüche 1 bis 4, **dadurch gekennzeichnet, dass** der Abstand zwischen dem Eintauchende des Messkopfes (2) und der an der seitlichen Umfangsfläche des Trägerrohres (1) oder des Messkopfes (2) angeordneten Einlauföffnung (4) kleiner als 50cm ist.

6. Vorrichtung nach einem der Ansprüche 1 bis 5, **dadurch gekennzeichnet, dass** der Abstand zwischen dem Eintauchende des Messkopfes (2) und der an der seitlichen Umfangsfläche des Trägerrohres (1) oder des Messkopfes (2) angeordneten Einlauföffnung (4) größer als 15 cm ist.

7. Vorrichtung nach einem der Ansprüche 1 bis 6, **dadurch gekennzeichnet, dass** das Volumen der Vorkammer (13) größer ist als das Volumen der Schlackeprobenkammer (16).

8. Vorrichtung nach einem der Ansprüche 1 bis 7, **dadurch gekennzeichnet, dass** der Durchmesser der an der seitlichen Umfangsfläche des Trägerrohres (1) angeordnete Einlauföffnung (4) in die Vorkammer (13) größer ist als der Durchmesser der in die Schlackeprobenkammer (16) mündenden Eingangsöffnung (15).

9. Vorrichtung nach einem der Ansprüche 1 bis 8, **dadurch gekennzeichnet, dass** die an der seitlichen Umfangsfläche des Trägerrohres (1) angeordnete Einlauföffnung (4) von einem brennbaren Material, insbesondere von Papier oder Pappe abgedeckt ist.

10. Vorrichtung nach einem der Ansprüche 1 bis 9, **dadurch gekennzeichnet, dass** die Schlackeprobenkammer (16) an ihrem der Vorkammer (13) zugewandten Ende und an ihrem der Vorkammer (13) abgewandten Ende von Metallplatten begrenzt ist.

11. Vorrichtung nach einem der Ansprüche 1 bis 10, **dadurch gekennzeichnet, dass** die Schlackeprobenkammer (16) zwischen ihrem der Vorkammer (13) zugewandten Ende und ihrem der Vorkammer (13) abgewandten Ende von einer konisch ausgebildeten Wand begrenzt ist.

## Claims

1. A device for measuring parameters or for taking samples in iron or steel melts as well as for sampling slag resting on an iron or steel melt, the device comprising an immersion end and a carrier pipe (1) having a lateral circumferential surface, with a measuring head (2) which has an immersion end and a lateral circumferential surface being arranged at the immersion end of the carrier pipe (1), wherein at least one sensor or an infeed opening (4) for a sample chamber (10) present in the interior of the device is arranged at the immersion end of the measuring head (2), wherein an infeed opening (4) is arranged on the lateral circumferential surface of the carrier pipe (1) or the measuring head (2), said infeed opening (4), via an infeed channel, ending in a prechamber (13) arranged in the interior of the carrier pipe (1) or the measuring head (2), and that the prechamber (13) has an inlet opening (15) at its end facing away from the immersion end of the measuring head (2), wherein said inlet opening (15) ends in a slag sample chamber (16) that is arranged in the interior of the device on the side of the prechamber (13) facing away from the immersion end,
**characterised in that**
a metallic splash protection layer (5) is arranged on the lateral circumferential surface of the carrier pipe (1) and the splash protection layer (5) surrounds the infeed opening (4) arranged on the lateral circumferential surface of the carrier pipe (1).

2. The device in accordance with Claim 1, **characterised in that** the splash protection layer (5) is designed in the form of a tube.

3. The device in accordance with Claim 1 or 2, **characterised in that** the splash protection layer (5) does not cover the infeed opening (4) arranged on the lateral circumferential surface of the carrier pipe (1).

4. The device in accordance with any one of Claims 1 to 3, **characterised in that** the splash protection layer (5) has a surface of at least 250 cm2, said surface facing away from the carrier pipe (1).

5. The device in accordance with any one of Claims 1 to 4, **characterised in that** the distance between the immersion end of the measuring head (2) and the infeed opening (4) arranged on the lateral circumferential surface of the carrier pipe (1) or the measuring head (2) is less than 50 cm.

6. The device in accordance with any one of Claims 1 to 5, **characterised in that** the distance between the immersion end of the measuring head (2) and the infeed opening (4) arranged on the lateral circumferential surface of the carrier pipe (1) or the measuring head (2) is greater than 15 cm.

7. The device in accordance with any one of Claims 1 to 6, **characterised in that** the volume of the prechamber (13) is greater than the volume of the slag sample chamber (16).

8. The device in accordance with any one of Claims 1 to 7, **characterised in that** the diameter of the infeed opening (4) that leads into the prechamber (13) and is arranged on the lateral circumferential surface of the carrier pipe (1) is greater than the diameter of the inlet opening (15) ending in the slag sample chamber (16).

9. The device in accordance with any one of Claims 1 to 8, **characterised in that** the infeed opening (4) arranged on the lateral circumferential surface of the carrier pipe (1) is covered by an inflammable material, more particularly by paper or cardboard.

10. The device in accordance with any one of Claims 1 to 9, **characterised in that** the slag sample chamber (16) is delimited by metal plates at its end facing the prechamber (13) and at its end facing away from the prechamber (13).

11. The device in accordance with any one of Claims 1 to 10, **characterised in that** the slag sample chamber (16) is delimited by a conically formed wall between its end facing the prechamber (13) and its end facing away from the prechamber (13).

## Revendications

1. Dispositif de mesure de paramètres ou de prélèvement d'échantillons dans des masses fondues de fer ou d'acier ainsi que de prélèvement d'échantillons de scorie reposant sur une masse fondue de fer ou d'acier, avec un tube porteur (1) présentant une extrémité d'immersion et une face périphérique latérale à l'extrémité d'immersion duquel une tête de mesure (2) présentant une extrémité d'immersion et une face périphérique latérale est disposée, dans lequel au moins un capteur ou une ouverture d'amenée (4) pour une chambre d'échantillons (10) présente à l'intérieur du dispositif est disposé(e) à l'extrémité d'immersion de la tête de mesure (2), dans lequel une ouverture d'amenée (4) est disposée sur la face périphérique latérale du tube porteur (1) ou de la tête de mesure (2), laquelle débouche par le biais d'un canal d'amenée dans une préchambre (13) disposée à l'intérieur du tube porteur (1) ou de la tête de mesure (2), et que la préchambre (13) présente à son extrémité détournée de l'extrémité d'immersion de la tête de mesure (2) une ouverture d'entrée (15) qui débouche dans une chambre d'échantillons de scorie (16) disposée à l'intérieur du dispositif sur le côté de la préchambre (13) détourné de l'extrémité d'immersion,
**caractérisé en ce que**
une couche protectrice contre les projections métalliques (5) est disposée sur la face périphérique latérale du tube porteur (1) et la couche protectrice contre les projections (5) entoure l'ouverture d'amenée (4) disposée sur la face périphérique latérale du tube porteur (1).

2. Dispositif selon la revendication 1, **caractérisé en ce que** la couche protectrice contre les projections (5) est réalisée de forme tubulaire.

3. Dispositif selon la revendication 1 ou 2, **caractérisé en ce que** la couche protectrice contre les projections (5) ne recouvre pas l'ouverture d'amenée (4) disposée sur la face périphérique latérale du tube porteur (1).

4. Dispositif selon une des revendications 1 à 3, **caractérisé en ce que** la couche protectrice contre les projections (5) présente une surface d'au moins 250 cm2 détournée du tube porteur (1).

5. Dispositif selon une des revendications 1 à 4, **caractérisé en ce que** l'écart entre l'extrémité d'immersion de la tête de mesure (2) et l'ouverture d'amenée (4) disposée sur la face périphérique latérale du tube porteur (1) ou de la tête de mesure (2) est inférieur à 50 cm.

6. Dispositif selon une des revendications 1 à 5, **caractérisé en ce que** l'écart entre l'extrémité d'immersion de la tête de mesure (2) et l'ouverture d'amenée (4) disposée sur la face périphérique latérale du tube porteur (1) ou de la tête de mesure (2) est supérieur à 15 cm.

7. Dispositif selon une des revendications 1 à 6, **caractérisé en ce que** le volume de la préchambre (13) est supérieur au volume de la chambre d'échantillons de scorie (16).

8. Dispositif selon une des revendications 1 à 7, **caractérisé en ce que** le diamètre de l'ouverture d'amenée (4) disposée sur la face périphérique latérale du tube porteur (1) dans la préchambre (13) est supérieur au diamètre de l'ouverture d'entrée (15) débouchant dans la chambre d'échantillons de scorie (16).

9. Dispositif selon une des revendications 1 à 8, **caractérisé en ce que** l'ouverture d'amenée (4) disposée sur la face périphérique latérale du tube porteur (1) est recouverte d'un matériau combustible, notamment de papier ou carton.

10. Dispositif selon une des revendications 1 à 9, **caractérisé en ce que** la chambre d'échantillons de scorie (16) est limitée à son extrémité tournée vers la préchambre (13) et à son extrémité détournée de la préchambre (13) par des plaques métalliques.

11. Dispositif selon une des revendications 1 à 10, **caractérisé en ce que** la chambre d'échantillons de scorie (16) est limitée entre son extrémité tournée vers la préchambre (13) et son extrémité détournée de la préchambre (13) par une paroi réalisée de manière conique.
